# EUROPEAN PATENT SPECIFICATION

(11) **EP 2 353 595 B1**
(45) Date of publication and mention of the grant of the patent: **28.10.2015**
(21) Application number: 10151114.5
(22) Date of filing: 19.01.2010
(51) Int. Cl.: A61K 31/202, A61P 3/06, A61P 3/10, A61P 3/04

(54) **Nutritional compensation for western-type diet**
Ernährungsausgleich für westliche Ernährungsweisen
Compensation nutritionnelle pour régime de type occidental

(43) Date of publication of application: 10.08.2011
(73) Proprietor: MJN U.S. Holdings LLC, Glenview, IL 60026 (US)
(72) Inventor: Harthoorn, Leunis Forrinus, 3446 AG Woerden (NL); Jouni, Zeina, Evansville IN, 47721 (US); van Tol, Eric Alexander Franciscus, 6824 MZ Arnhem (NL); Kleemann, Robert, 2333 CK Leiden (NL); Kooistra, Taeke, 2333 CK Leiden (NL)
(74) Representative: Hatzmann, Martin

(56) References cited:
- US-A1- 2007 203 237
- US-A1- 2007 203 238
- US-A1- 2008 269 328
- LINA B A R ET AL: "Subchronic (13-week) oral toxicity study, preceded by an in utero exposure phase, with arachidonate-enriched triglyceride oil (SUNTGA40S) in rats" FOOD AND CHEMICAL TOXICOLOGY, PERGAMON, GB LNKD- DOI:10.1016/J.FCT.2005.08.002, vol. 44, no. 3, 1 March 2006 (2006-03-01), pages 326-335, XP025065441 ISSN: 0278-6915 [retrieved on 2006-03-01]
- RUZICKOVA JANA ET AL: "Omega-3 PUFA of marine origin limit diet-induced obesity in mice by reducing cellularity of adipose tissue" LIPIDS, vol. 39, no. 12, December 2004 (2004-12), pages 1177-1185, XP002585444 ISSN: 0024-4201
- Alison M Hill ET AL: "Combining fish-oil supplements with regular aerobic exercise improves body composition and cardiovascular disease risk factors 1-3", , 1 May 2007 (2007-05-01), XP055138972, Retrieved from the Internet: URL:http://ajcn.nutrition.org/content/85/5 /1267.full.pdf [retrieved on 2014-09-09]

## Description

### Field of the invention

The invention relates to the nutritional or pharmaceutical intervention in compensating for adverse health effects of diets rich in fats and carbohydrates, such as typically found in western societies, and other developed areas of the world. Particularly, the invention pertains to the use of a nutritional supplement for this purpose, and to a nutritional composition, particularly a nutritional supplement for use in the prevention and treatment of said adverse health effects.

### Background of the invention

Diets rich in fats and carbohydrates are increasingly being consumed by many populations in the world. This is particularly the case in western societies, such as the US and the European Union, and in other developed countries throughout the world.

These diets themselves are increasingly associated with conditions such as overweight, and various health problems associated therewith, such as cardiovascular diseases, mobility disorders, type-2 diabetes mellitus, and a variety of other related disturbances or disorders. (References: Bray 2004; Parillo & Riccardi 2005; Cordain et al, 2005).

Also the Western-type high-fat high-sugar diet is frequently considered to relate to specific health conditions such as dyslipidemia, i.e. hypercholesterolemia, hypertriglyceridemia and hyperlipidemia, and impaired glucose tolerance and insulin-resistance. The effects of the diet *per se are* generally considered to be worsened by a general sedentary lifestyle and lack of physical exercise that large populations in the same societies are prone to.

Many solutions have been proposed, ranging from suggesting various dietary rules so as to avoid or reduce the high fat, high carbohydrate intake, to guided physical exercise methods. These methods generally suffer from serious compliance problems, or at least require great efforts to secure compliance. Also, following dietary rules (of which a wide variety of different teachings exist), generally leads to "yo-yo" effects, caused when people lose body weight by following a dietary rule for a limited period of time, and then return to their original, Western-type high-fat, high-sugar diet or intake pattern. After that they will generally put on body weight again, and frequently even end-up at a higher body weight than before starting to lose weight. Also, a large variety of dietary products (replacements or supplements) are available with the aim to help people losing body weight. These slimming products generally serve to induce a feeling of satiety, so as to reduce the intake of food. These products incur the risk that people using them may not receive a diet that is nutritionally complete.

US 2007/0203237 addresses the administration, of a therapeutically effective amount, of docosahexaenoic acid (DHA) and/or arachidonic acid (ARA) for preventing or treating obesity in a subject. To this end an amount of 15-60 mg of DHA per kg bodyweight and 20-60 mg per kg bodyweight of ARA is administered daily. The effect of the DHA/ARA as described, is to increase the amount of lean muscle in the body, and to decrease the amount of adipose tissue. The subjects generally range in age from 0 (infants) to 12 years. The described method is tested in baboon neonates.

Similarly, US 2007/0026049 refers to the administration of DHA and ARA in infants, particularly preterm infants, with the aim to increase lean body mass and reduce fat body mass.

Another disclosure in this area is US 2007/0203238, which refers to a method of reducing triglyceride levels in a subject, particularly an infant through ARA / DHA supplementation. The purpose hereof relates to reducing the likelihood of triglyceride-linked diseases and disorders later in life. Typically, the method aims at a population that is on infant formulae, and it does not refer to effects of ARA / DHA in combination with high-fat high-sugar diets. The method is tested in baboon neonates.

US 2004/0132819 (and WO 2004/012727) describes products containing long-chain polyunsaturated fatty acids (LC-PUFAs), particularly omega-3 fatty acids such as DHA, to control appetite and to help treat and/or prevent obesity and conditions of overweight, particularly in a pediatric population. The reference focuses on the effects of certain components on food intake regulation. A particularly described effect is that post-weaning feeding with ARA intake, regardless of the DHA level, results in increased food intake, whilst post-weaning feeding with DHA, regardless of the ARA level, results in reduced food intake. On this basis, the reference presents the use of omega-3 LC-PUFAs such as DHA to decrease the appetite of a mammal. The present invention, however, is starts *inter alia* from the acknowledgement that appetite suppression *per se* may not be a healthy way of provoking body weight loss, just as with the inducement of satiety by the aforementioned slimming products.

Other references, seek to prevent obesity and other, related, disorders later in life, by administering certain compositions to infants. Thus, e.g., WO 2007/073194, presents a composition having a lipid fraction enriched in glycerophospholipids, sphingolipids and cholesterol, for administration to non-obese infants below 3 years, with the aim to prevent obesity later in life. Further, e.g., WO 2008/054208 presents certain lipids-containing compositions, *inter alia* based on DHA and ARA, for administration to infants between 0 and 36 months of age, with the aim to prevent and/or treat visceral adiposity and/or the accumulation of visceral fat mass to an excessive amount.

It should be noted that overweight does not always imply obesity. A person is generally considered overweight on the basis of a BMI (body mass index) of over 25, and obese on the basis of a BMI of over 30. A treatment of obesity does not always imply a treatment of overweight. On the other hand, overweight may be a pre-stage of obesity and it is therefore of potential importance to reduce weight at the stage of overweight.

In EP 1 886 680 it is recognized that in the developed world, at least, there are increasing concerns over the physiological effects of a diet rich in saturated fats and favouring the precursors of omega-6 LC-PUFAs such as ARA, at the expense of the precursors of omega-3 LC-PUFA, such as DHA, on the physiological omega-6: omega-3 ratio in general. The reference particularly acknowledges concerns regarding pregnant women, and proposes to administer particularly DHA to pregnant or breast-feeding women, with the aim to exert the beneficial effects thereof to the foetus or infant.

Lina et al., Food and Chemical Toxicology (2006), vol.44 no.3, pages 326-355, discloses an oral toxicity study in rats. As part of this study, cholesterol levels are determined after the administration of a "high fat" diet having an increased amount of 5.76 wt.% of fat, in comparison with a low fat diet. Reduced cholesterol levels are measured in the event that an oil mixture comprising ARA and DHA is used in replacement of corn oil, in the "high fat" diet.

With the present invention, rather than replacing fat in a diet, it is desired to provide a composition that helps to counteract the effects of the above-mentioned high-fat high-sugar diet. It will be understood that this is of relevance to populations that are prone to take a high-fat high-sugar diet, i.e. not infants or small children that are on breast-feeding, on a formula, or on dedicated childrens' nutrition.

It would be advantageous, if a nutritional composition were provided that aids in counteracting the effects of a Western-type high-fat high-sugar diet, without having to change the diet, or to take products that induce satiety or reduce appetite.

### Summary of the Invention

In order to better address one or more of the foregoing desires, the invention, in one aspect, presents a composition comprising docosahexaenoic acid (DHA) and arachidonic acid (ARA), for use in compensating for hyperchlesterolemia in a human subject, wherein the human subject is on a diet having between 25-40 wt% fat, 40-55 wt% carbohydrates and 10-20 wt% protein and whereinthe composition is administered as a daily supplement to said diet.

### Detailed Description of the Invention

The invention is concerned with the adverse health effects of a 'Western-type' diet, also recognized as a 'high-fat high-sugar' diet.

The Western-type high-fat high-sugar diet is generally characterized by a high intake of processed meat, red meat, butter, high-fat dairy products, eggs, sugar and refined grains and is a common dietary habit in many developed countries and an increasing dietary pattern in developing countries.

In terms of food quality, the Western-type high-fat high-sugar diet can more specifically be characterized by its high intake of saturated, trans- and omega-6 fatty acids, low content of omega-3 fatty acids, high amount of easily-digestible sugars (i.e. mono- and disaccharides) and refined grain products, and low fibre and antioxidants intake (Hu & Willett, 2002; Parillo & Riccardi, 2002; Mente et al., 2009).

These dietary characteristics promote a high glycemic response and unfavorable blood lipid profiles (Jenkins et al., 1987) and have been associated witch an increased risk for type-2 diabetes mellitus, dyslipidemia, cardiovascular diseases (e.g. coronary heart failure), overweight, obesity and obesity-related disturbances (e.g. inflammatory responses) (Bray et. al., 2004; Gross et al., 2004; Giugliano et al.,2006; Lairon, 2008), including childhood overweight (Ailhaud et al., 2008).

A lower cardiovascular risk profile in humans was found with lower insulin resistance (Smiley et al, 2001) and when LDL-cholesterol was substantially reduced and the HDL-cholesterol increased by more than 7.5% (Cui et al, 2009). Herein HDL stands for "high density lipoprotein" which is generally known as "good" cholesterol, and LDL stands for "low density lipoprotein" which is generally known as "bad" cholesterol.

The opposite of the Western-type high-fat high-sugar diet is either a 'Mediterranean-type' or 'prudent' diet containing higher amounts of (non-hydrogenated) unsaturated fats as the predominant form of dietary fat, whole grains as the main form of carbohydrates, an abundance of fruits and vegetables, and adequate omega-3 fatty acids, and which have been suggested to reduce the health risks of cardiovascular disease and obesity (Hu & Willett, 2002).

The aforementioned references are:
Ailhaud G, Guesnet P, Cunnane SC. An emerging risk factor for obesity: does disequilibrium of polyunsaturated fatty acid metabolism contribute to excessive adipose tissue development? Br J Nutr. 2008, 100, 461-470;
Bray GA, Nielsen SJ, Popkin BM. Consumption of high-fructose corn syrup in beverages may play a role in the epidemic of obesity. Am J Clin Nutr. 2004, 79, 537-543;
Cui Y, Watson DJ, German CJ, Shapiro DR, Gotto AM, Hiserote P, Clearfield MB. Effects of increasing high-density lipoprotein cholesterol and decreasing low-density lipoprotein cholesterol on the incidence of first acute coronary events (from the Air Force/Texas Coronary Atherosclerosis Prevention Study). Am J Cardiol. 2009, 104, 829-834.
Giugliano D, Ceriello A, Esposito K. The effects of diet on inflammation: emphasis on the metabolic syndrome. J Am Coll Cardiol. 2006, 48, 677-685;
Gross LS, Li L, Ford ES, Liu S. Increased consumption of refined carbohydrates and the epidemic of type 2 diabetes in the United States: an ecologic assessment. Am J Clin Nutr. 2004, 79, 774-779;
Hu FB, Willett WC. Optimal diets for prevention of coronary heart disease. JAMA. 2002, 288, 2569-2578;
Jenkins DJ, Jenkins AL, Wolever TM, Collier GR, Rao AV, Thompson LU. Starchy foods and fiber: reduced rate of digestion and improved carbohydrate metabolism. Scand J Gastroenterol. Suppl. 1987, 129, 32-141;
Lairon D. Macronutrient intake and modulation on chylomicron production and clearance. Atheroscler Suppl. 2008, 9, 45-48;
Mente A, de Koning L, Shannon HS, Anand SS. A systematic review of the evidence supporting a causal link between dietary factors and coronary heart disease. Arch Intern Med. 2009, 169, 659-669;
Parillo M, Riccardi G. Diet composition and the risk of type 2 diabetes: epidemiological and clinical evidence. Br J Nutr. 2004, 92, 7-19;
Smiley T, Oh P, Shane LG. The relationship of insulin resistance measured by reliable indexes to coronary artery disease risk factors and outcomes--a systematic review. Can J Cardiol. 2001, 17, 797-805.

It is to be emphasized that, whilst the terms "high-fat" and "high-sugar" (or "high-carbohydrate") are by nature relative, the person skilled in the art is well aware of whether or not a diet can be characterized as a Western-type diet. More specifically, the more abundant form of the Western-type high-fat high-sugar diet is quantitatively characterized by a high caloric / energy density (e.g. high glycemic load) and contains on average 25-40 wt% fat, 40-55 wt% carbohydrates and 10-20 wt% protein Cordain 2005). From a qualitative perspective, the Western-type high-fat high-sugar diet is often characterized by a high cholesterol, trans- and saturated fatty acid content and a high content of refined grains (>20 en%), refined sugars (>20 en%) as well as refined fats (>20 en%), accompanied with low omega-3 fatty acid content (Cordain, 2005). A specific form of the Western-type diet is recognized in the art as the "Standard American Diet" (SAD).

The invention is generally based on the novel and unexpected finding that ARA and/or DHA dietary supplementation, on top of a Western-type high-fat high-sugar diet, is capable of reducing cholesterol.

Particularly, the composition was found to be capable of providing a healthier plasma cholesterol profile and/or lower blood glucose.

The invention makes use of a mixture of ARA and DHA, hereinafter referred to as the composition of the invention.

If the composition of the invention comprises a mixture, this has a ratio, by weight, of ARA:DHA typically from about 1:3 to about 9:1. In one embodiment of the present invention, this ratio is from about 1:2 to about 4:1. In yet another embodiment, the ratio is from about 1: 1.5 to about 2:1. In one particular embodiment the ratio is about 2:1. In another particular embodiment of the invention, the ratio is about 1 :1.5. In other embodiments, the ratio is about 1 :1.3. In still other embodiments, the ratio is about 1 :1.9. In a particular embodiment, the ratio is about 1.5:1. In a further embodiment, the ratio is about 1.47:1.

Preferably, the ARA and DHA are used in effective amounts of at least 129mg/100g food (ARA) and at least 88 mg/100g food for DHA, or at least 25 mg/100kcal for ARA and at least 17 mg/100 kcal for DHA.

By further preference, the composition of the invention is generally used so as to provide ARA in an amount of from 25 mg to 40 mg per 100 kcal food and/or DHA in an amount of from 15 mg to 60 mg per 100 kcal food. If the composition of the invention is a nutritionally complete formula, the foregoing amounts are per 100 kcal of such formula.

Preferred daily dosages of ARA and DHA are 50 to 750 mg/day for ARA and 50 to 450 mg/day for DHA.

The DHA and ARA can be presented in any form suitable for oral or otherwise enteral administration to a human subject. Thus, e.g., the DHA. and ARA can be administered via oral dosage units, such as oral pharmaceutical dosage units, including but not limited to tablets, pills, capsules, caplets, gelcaps, oil drops, sachets, effervescent formulations, and powders.

Particularly, the composition of the invention is presented in the form of a dietetic or nutritional composition, suitable for human consumption. In a specific embodiment hereof, the DHA and ARA are presented as a nutritional supplement. Such a supplement can be in any form suitable for addition to foods and/or drinks, including but not limited to powders, granules, oils, oil-in-water emulsions, and pastes. In yet a further embodiment, the DHA and ARA are pre-added to a food or drink product. Thus, e.g., the ARA and DHA can be added to a dairy drink such as milk, a milk powder formulation, or yoghurt, to a dessert such as pudding, to a fruit drink, to instant soup formulations, or to snacks such as muesli bars, candy bars, and the like.

Whilst the foregoing generally refers to oral forms of administration of the composition used in the present invention, the DHA and ARA can also be administered via any other enteral route of administration.

In one embodiment, the composition of the invention is given for the nutritional purpose of compensating for hypercholesterolemia as an adverse health effects of the aforementioned Western-type high-fat high-sugar diet. Herein the term "compensating" will generally be understood to have its normal meaning, viz. it refers to offsetting or counterbalancing. The term "compensating' or, for that matter, "counterbalancing" is by no means intended to refer to a full compensation or counterbalance, but preferably this contribution amounts to a full compensation.

The DHA and ARA according to the invention can be presented separately, preferably with an instruction to take them on the same day, and more preferably at about the same time. This form of presentation, i.e. separate formulations intended for simultaneous daily use, for ease of reading, is also considered hereinafter as a composition of the invention. Preferably, the ARA and DHA are present in one and the same formulation, i.e. present in one and the same nutritional supplement or food or drink.

In yet another embodiment, the DHA and ARA are presented in a formula intended for children such as "growing-up milk" typically aiming at children of 3 to 12 years' old. Growing-up milks, and their general composition, are known to the skilled person, and do not require elucidation here.

In some embodiments, the DHA and ARA are presented in formulations that are nutritionally complete, and which contain suitable types and amounts of lipid, carbohydrate, protein, vitamins and minerals. The invention does not, however, aim at substituting such formulations for normal food. Rather, as opposed to products intended to reduce appetite or other slimming products as described above, the invention expressly aims at allowing subjects that are on a Western-type high-fat high-sugar diet, to not change their diet, and yet be less prone to the aforementioned health consequences of such a diet.

The source of the ARA and DHA can be any source known in the art such as marine oil, fish oil, single cell oil, vegetable oil, egg yolk lipid, brain lipid, and the like. The DHA and ARA can be in natural form. Alternatively, the DHA and ARA can be used in refined form. Sources of DHA and ARA may be single cell oils as taught in U.S. Pat. Nos. 5,374,657, 5,550,156, and 5,397,591.

The invention concerns the administration of the aforementioned composition comprising DHA and ARA to a human subject that is on a Western-type high-fat high-sugar diet. The expression "to be on a diet" in the context of the invention means that the subject generally eats on one day a week, particularly on three to five days a week, and more particularly on six to seven days a week, one or more meals that lead to the intake of levels of saturated fats and refined sugars commensurate with the common understanding in the art of the term Western-type high-fat high-sugar diet. The foregoing does not imply that this would hold for every week, but could also be the average food intake when analyzed over a period of a month, of two months, or of a longer period such as one or more quarters, trimesters, semesters, or years.

In a preferred embodiment, the invention more expressly relates to a direct contribution to the compensation of one or more health effects of a Western type high-fat high-sugar diet, by presenting a dietary regimen to a subject that includes one dosage of the ARA and DHA composition described above, for each day that the subject had a food intake in accordance with the Western-type high-fat high-sugar diet. More preferably, the composition is used as a daily nutritional supplement for a human subject during each week that said subject on one or more days had a food intake in accordance with the Western-type high-fat high-sugar diet. Most preferably, the composition of the invention is presented for permanent daily use by a subject that is on a Western-type high-fat high-sugar diet on a permanent basis. Herein "permanent" refers to an undefined period of time, and does not exclude one or more days that either the diet may be different (e.g. a subject occasionally takes a Mediterranean meal, another non-Western type high fat / high sugar diet such as a Japanese meal, or prudent diet), or the nutritional supplement may not be taken.

For use in compensating for hypercholesterolemia as an adverse health effects of a Western-type high-fat high-sugar diet, the composition of the invention is generally taken in the above-mentioned amounts effective amounts of at least 129mg/100g food (ARA) and at least 88 mg/100g food for DHA, or at least 25 mg/100 kcal food of ARA and 17 mg/100 kcal food for DHA. The daily doses are strongly dependent on age and body weight, and preferably are between 50 mg/day of DHA and 50 mg/day of ARA for an infant (e.g. 6 months of age) to 1000 mg/day of DHA and 1400 mg/day of ARA for an adult human subject, and more preferably between 50 mg/day of DHA and 50 mg/day of ARA for an infant (e.g. 6 months old) to 450 mg/day of DHA and 650 mg/day of ARA for an adult woman and 550 mg/day of DHA and 750 mg/day of ARA for an adult man.

The composition for use of the invention contributes to compensating the plasma cholesterol profile of the subject taking the composition. The life long intake of the composition also serves to provide healthier cholesterol levels, i.e. an increased HDL/LDL ratio and a reduced VLDL-cholesterol level. Herein HDL and LDL have the previously given meaning. VLDL stands for "very low density lipoprotein."

In a particularly preferred embodiment, the composition of the invention is used in children of from 3 years old (36 months) to 12 years old.

It is to be understood that the invention is not limited to the embodiments as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

The invention will be illustrated with reference to the following, non-limiting Example and the accompanying non-limiting Figures.

### Description of the Drawings

Fig. 1. (reference) Body weight development of ApoE3L-mice fed a high-fat high-sugar diet. ARIA / DHA dietary supplementation significantly reduced body weight over time (P<0.01, ANOVA repeated measures).
Fig. 2. Plasma total cholesterol (mM) in ApoE3L-mice fed a high-fat high-sugar diet. ARA / DHA dietary supplementation significantly reduced total cholesterol over time (P<0.01, ANOVA repeated measures).
Fig 3. AKTA-FPLC-differentiation of (a) HDL- and LDL-cholesterol and (b) VLDL-cholesterol (mainly triglycerides) (mM) in plasma of ApoE3L-mice (pooled) after feeding a high-fat high-sugar diet for 16 weeks. ARA / DHA dietary supplementation reduced LDL- and VLDL-cholesterol (triglycerides) and increased HDL-cholesterol (inserted bar diagram: ARA / DHA increased the HDL/LDL ratio by about 22%).
Fig. 4. (reference) Plasma glucose (mM) after a 4-hours fasting period in ApoE3L-mice fed a high-fat high-sugar diet. ARA / DHA dietary supplementation significantly reduced fasting glucose plasma levels over time (P=0.05, ANOVA repeated measures).
Fig. 5 (reference) Mean liver weight (relative to body weight) of ApoE3L-mice at 16 weeks. ARA / DHA supplementation (stripes) showed a significant decrease in relative liver weight (P=0.01, t-test) in comparison to a high-fat high-sugar diet (black).
Fig. 6. Plasma (reference) ALAT (U/l) (alanaine aminotransferase) of ApoE3L-mice at 16 weeks. In comparison to a high-fat high-sugar diet (control, black) ARA / DHA supplementation (stripes) tended top reduce ALAT (P = 0.097, t-test).

### Example

### Test Description

ARIA and DHA (as currently present in infant formulae) were tested for possible beneficial effects on body weight development, dyslipidemia and insulin-resistance underlying overweight and obesity in ApoE3L-mice. These mice are a well-established humanized animal model for hyperlipidemia with overweight and mild obesity and insulin-resistance.

In the test, the ARA / DHA mixture in amounts of 0.129 %w/w and 0.088%w/w, respectively (ratio 1.5:1) was added to a high-fat high-sugar diet, containing 12.2 %w/w saturated fatty acids, 9.67 %w/w monosaturated fatty acids, 5.67 %w/w linoleic acid (LA), 0.63 %w/w alpha-linolenic acid (ALA), and 42 %w/w carbohydrates, for being fed to ApoE3L-mice. The following characteristics were measured during the course of a dietary period of 16 weeks: food intake, body weight, plasma total cholesterol, total triglycerides, HDL-cholesterol, LDL-cholesterol, VLDL-cholesterol (triglycerides), fasting plasma glucose and alanine-aminotranferase (ALAT) (biomarker of liver physiology). At the end of the experiment (16 weeks) weights of different types of fat tissues (gonadal, subcutaneous, visceral and brown) and liver were determined. The results of this study show (as a reference) that ARA / DHA dietary supplementation, on top of a high-fat high-sugar diet, significantly lowers body weight in ApoE3L-mice as compared to the control group with the high-fat high-sugar (including LA and ALA) diet only. The ARA / DHA containing diet did not affect food intake. In accordance with the invention, the ARA / DHA dietary supplementation reduced plasma levels of total cholesterol and VLDL (triglycerides), and increased HDL/LDL-ratio. As a reference, ARA / DHA also significantly reduced relative liver weight at 16 weeks of age, while no effects were found at the different fat-tissue weights. The ARA / DHA mixture was able, also presented by way of reference, to decrease fasting plasma glucose levels.

### Results:

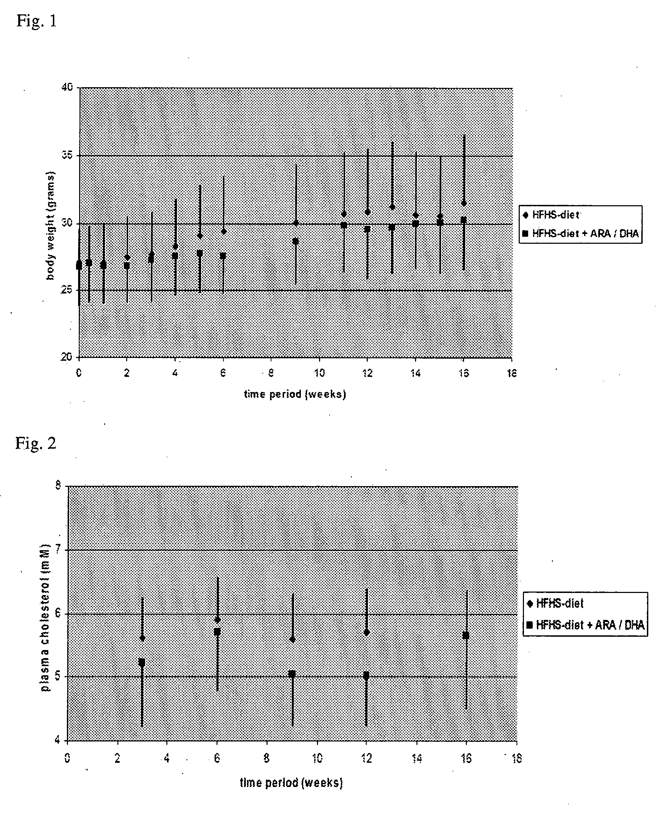

The ARA / DHA supplementation to the high-fat high-sugar diet in ApoE3L-mice showed a significantly lower body weight over time as compared to their high-fat / carbohydrate-rich (including LA and ALA) control group (Fig. 1) (P<0.01).

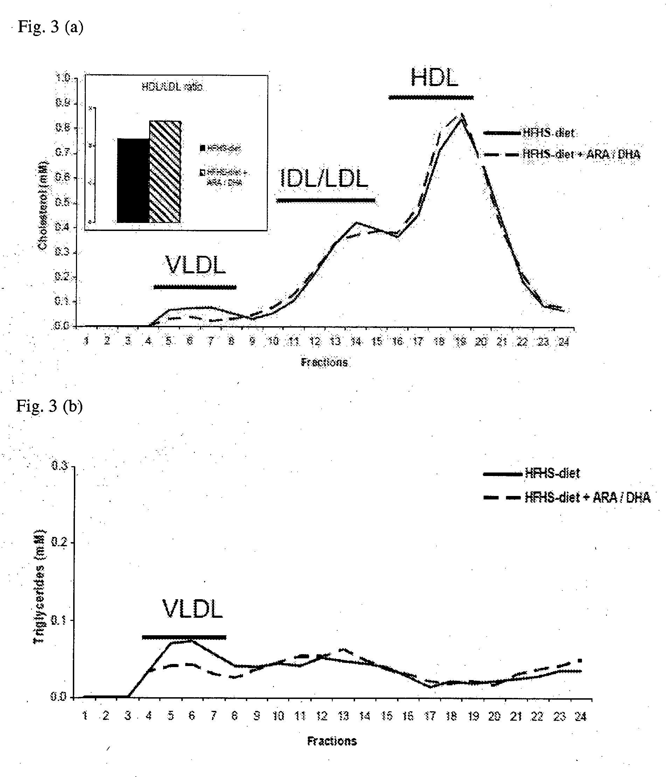

After 6 weeks, mice with the ARA / DHA supplementation had about 10% lower body weight than the control group with the high-fat high-sugar diet only, and after 16 weeks of dietary intervention the ARA / DHA fed mice still stayed at a lower body weight of about 5% in comparison to the control mice. Plasma levels of total cholesterol were significantly lower in the ARA / DHA supplemented group than in the high-fat / high-sugar control group (Fig. 2) (P<0.01). No ageing effect was found. At 9 and 12 weeks the reductions in total cholesterol were about 10%. Plasma levels of total triglycerides did not differ between the ARA / DHA treatment and the control group however at the differentiated level after Fast Protein Liquid Chromatography (FPLC) separation of pooled plasma samples (ARA / DHA-treated group vs. control group) by means of AKTA-analysis at 16 weeks, differences were found both at the cholesterol and triglyceride profiles (Fig. 3 A, B). ARA / DHA supplementation decreased the amount of VLDL- and LDL-cholesterol particles in comparison to the pooled plasma samples of the high-fat high-sugar control group (Fig. 3(a), while HDL-cholesterol levels were higher in the ARA / DHA supplemented group. This resulted in a HDL / LDL-ratio of 2.667 in the ARA / DHA treated group vs. 2.181 in the high-fat high-sugar control group. The VLDL-profiles at the triglycerides level showed a strong reduction by ARA / DHA intake (Fig. 3(b)).

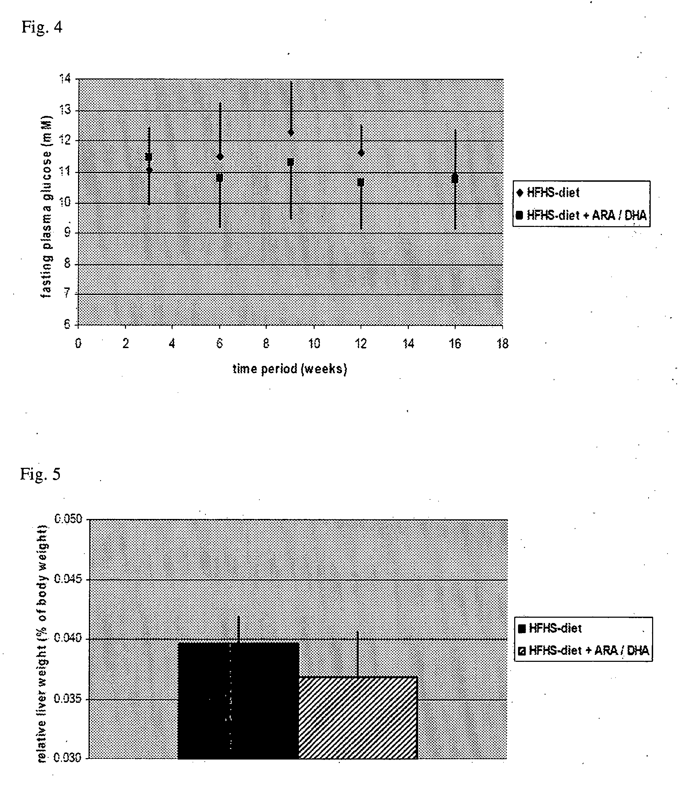

Plasma glucose levels were measured at 3, 6, 9 and 12 weeks after a 4-hours fasting period at each time point. The ARA / DHA supplemented ApoE3L-mice demonstrated a lower plasma glucose than the high-fat high-sugar control group (Fig. 4) (P=0.05). Especially at 3, 6, and 9 weeks, fasting plasma glucose levels were 6 - 8 % lower (Fig. 4).

Relative liver weights, expressed as liver weight per body weight, or the ARA / DHA supplemented group were about 7 % lower than in the high-fat high-sugar control group (P=0.01) (Fig. 5). This suggests that the high fat high-sugar diet induces fat accumulation in the liver (steatosis).

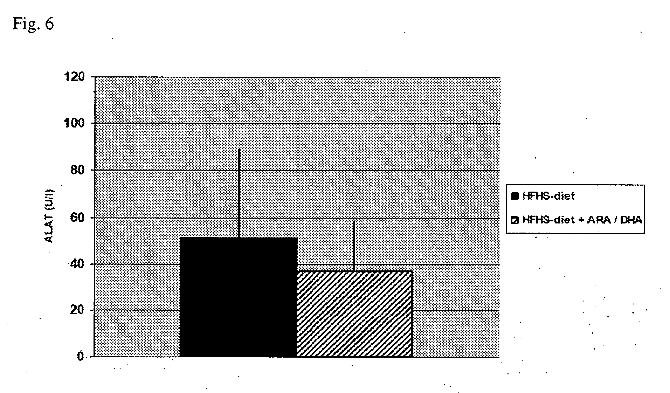

This was confirmed by the liver physiology marker ALAT (alanine amino transferase), showing the same pattern (Fig. 6). The ARA / DHA supplementation to the high-fat high-sugar diet tended to reduce ALAT.

### Conclusion:

Under high-fat high-sugar dietary conditions, ARA / DHA dietary supplementation (0.129 w/w % and 0.088 w/w %, respectively) (ratio 1.5:1) is able to reduce body weight in ApoE3L-mice by 5 to 10%. ARA/DHA supplementation showed no changes in food intake.

ARA DHA. dietary supplementation has beneficial effects on the development of dyslipidemia in three ways, 1: it reduces hypercholesterolemia characterized by lower plasma levels of both total cholesterol and LDL-cholesterol, 2: it reduces plasma VLDL-cholesterol levels and increases the HDL/LDL ratio, and 3: it reduces hyperlipidemia shown by both a lower LDL and VLDL profile.

## Claims

1. A composition comprising docosahexaenoic acid (DHA) and arachidonic acid (ARA) for use in compensating for hypercholesterolemia in a human subject, wherein the human subject is on a diet having between 25-40 wt% fat, 40-55 wt% carbohydrates and 10-20 wt % protein and wherein the composition is administered as a daily supplement to said diet.

2. A composition for use according to claim 1, wherein the daily dosage of ARA is 50 to 750 mg and the daily dosage of DHA is 50 to 450 mg.

3. A composition for use according to any one of the preceding claims, wherein ARA and DHA are administered in a ratio of from 1:3 to 9:1, preferably 1:2 to 4:1.

4. A composition for use according to any one of the preceding claims, wherein DHA and ARA are administered a single composition, preferably selected from the group of tablets, pills, capsules, caplets, gel caps, oil drops, sachets, effervescent formulations, and powders.

5. A composition for use according to any one of the claims 1 to 4, wherein the composition is provided in a food or drink product.

## Patentansprüche

1. Zusammensetzung, umfassend Docosahexaensäure (DHA) und Arachidonsäure (ARA) zur Verwendung beim Ausgleich von Hypercholesterinämie bei einem menschlichen Patienten, wobei der menschliche Patient auf einer Diät mit zwischen 25-40 Gew.-% Fett, 40-55 Gew.-% Kohlehydraten und 10-20 Gew.-% Protein ist und wobei die Zusammensetzung als eine tägliche Ergänzung zu der Diät verabreicht wird.

2. Zusammensetzung zur Verwendung nach Anspruch 1, wobei die tägliche Dosis ARA 50 bis 750 mg und die tägliche Dosis DHA 50 bis 450 mg ist.

3. Zusammensetzung zur Verwendung nach einem der vorhergehenden Ansprüche, wobei ARA und DHA in einem Verhältnis von 1:3 bis 9:1, bevorzugt 1:2 bis 4:1 verabreicht werden.

4. Verfahren zur Verwendung nach einem der vorhergehenden Ansprüche, wobei DHA und ARA in einer einzigen Zusammensetzung verabreicht werden, bevorzugt ausgewählt aus der Gruppe von Tabletten, Pillen, Kapseln, Filmtabletten, Gelkapseln, Öltropfen, Tütchen, Brausetablettenformulierung und Pulvern.

5. Zusammensetzung nach einem der Ansprüche 1 bis 4, wobei die Zusammensetzung in einem Lebensmittel- oder Getränkeprodukt bereitgestellt ist.

## Revendications

1. Une composition comprenant de l'acide docosahexaenoïque (DHA) et de l'acide arachidonique (ARA), pour une utilisation pour compenser l'hypercholestérolémie d'un sujet humain, dans laquelle utilisation le sujet humain est soumis à un régime impliquant entre 25-40% en poids de matière grasse, 40- 55 % en poids de carbohydrates et 10-20% en poids de protéine, et dans laquelle utilisation la composition est administrée en complément quotidien dudit régime.

2. Une composition pour une utilisation selon la revendication 1, dans laquelle utilisation la dose quotidienne de ARA est de 50 à 750 mg et la dose quotidienne de DHA est de 50 à 450 mg.

3. Une composition pour une utilisation selon l'une quelconque des revendications précédentes, dans laquelle utilisation ARA et DRA sont administrés en un rapport de 1/3 à 9/1, de préférence de 1/2 à 4/1.

4. Une composition pour une utilisation selon l'une quelconque des revendications précédentes, dans laquelle utilisation DHA et ARA sont administrés dans une composition unique, de préférence choisie dans le groupe formé par les comprimés, les pilules, les capsules, les caplets, les capsules de gels, les émulsions huileuses, des sachets, des formulations effervescentes et des poudres.

5. Une composition pour une utilisation selon une quelconque des revendications 1 à 4, dans laquelle utilisation la composition est fournie dans un produit alimentaire ou une boisson.
